# EUROPEAN PATENT SPECIFICATION

(11) **EP 3 888 595 B1**
(45) Date of publication and mention of the grant of the patent: **15.07.2026**
(21) Application number: 21168724.9
(22) Date of filing: 07.06.2018
(51) Int. Cl.: A61F 2/16, A61F 9/00, A61F 9/007, G01M 11/00

(54) **INTRAOCULAR PSEUDOPHAKIC CONTACT LENS WITH MECHANISM FOR SECURING BY ANTERIOR LEAFLET OF CAPSULAR WALL AND RELATED SYSTEM**
INTRAOKULARE PSEUDOPHAKISCHE KONTAKTLINSE MIT MECHANISMUS ZUR BEFESTIGUNG DURCH ANTERIORE SCHICHT EINER KAPSELWAND UND ZUGEHÖRIGES SYSTEM
LENTILLE DE CONTACT INTRAOCULAIRE PSEUDOPHAQUE AVEC MÉCANISME DE FIXATION PAR FEUILLET ANTÉRIEUR DE PAROI CAPSULAIRE ET SYSTÈME

(30) Priority: 11.07.2017 US 201715646254
(43) Date of publication of application: 06.10.2021
(62) Divisional of application: 18831320.9
(73) Proprietor: Onpoint Vision, Inc., Aliso Viejo CA 92656 (US)
(72) Inventor: Cady, Kevin J., St.Charles, IL 60175 (US)
(74) Representative: Barker Brettell LLP

(56) References cited:
- WO-A1-2015/026226
- US-A1- 2015 342 729
- US-A1- 2016 081 791
- US-A1- 2016 317 286

## Description

### TECHNICAL FIELD

This disclosure relates generally to implantable optical devices. More specifically, this disclosure relates to an intraocular pseudophakic contact lens with a mechanism for securing by an anterior leaflet of a capsular wall and a related system and method.

### BACKGROUND

In a normal human eye, light enters through the cornea and passes through the pupil, and the natural crystalline lens focuses the light onto the retina of the eye. However, due to cataracts or other problems, the natural crystalline lens of an eye may need to be replaced with an artificial intraocular lens (IOL). The term "pseudophakia" is used to describe an eye in which the natural crystalline lens has been replaced with an intraocular lens.

Before an intraocular lens is placed into a patient's eye, a doctor or other personnel typically selects an intraocular lens that is designed to provide desired refractive correction for the patient's eye. For example, an intraocular lens could have an optical lens designed to correct myopia (near-sightedness), hyperopia (far-sightedness), astigmatism, or other refractive errors that occur naturally in the patient's eye. However, it is often the case that the intraocular lens selected for a patient's eye does not fully correct (and may even cause) some form of refractive error in the patient's eye. This refractive error is referred to as "residual" refractive error.

There are various conventional options for correcting residual refractive error, all of which have their disadvantages. For example, one intraocular lens in a patient's eye could be replaced with a different intraocular lens, but this typically has a high risk of surgical complications. Ablation surgery (such as LASIK) on the cornea of a patient's eye could be done to correct residual refractive error, but this can have a high level of unwanted side effects, particularly for older patients. An additional intraocular lens (often referred to as a "piggyback" IOL) could be inserted in front of an existing intraocular lens, but this is typically an invasive procedure with less predictability associated with the final refractive outcome. In addition, intracorneal lenses (ICLs) can be inserted into the cornea of a patient's eye, but this is often more invasive and has a high degree of rejection. In general, the above procedures are typically not predictable and have a higher degree of surgical risk. Also, the devices used in the above procedures are difficult to remove and "reverse" any residual refractive error, resulting in a higher risk of leaving the patient with induced visual aberration.

Document US-A-2016/317286 discloses a two-part accommodating intraocular lens (IOL) device for implantation in a capsular bag of a patient's eye. The IOL device includes a primary lens assembly and a power changing lens. The primary lens assembly includes a fixed lens and a peripherally disposed centration member. The centration member has a circumferential distal edge and a first coupling surface adjacent the circumferential distal edge. The power changing lens has an enclosed, fluid- or gel-filled lens cavity and haptic system disposed peripherally of the lens cavity. The haptic system has a peripheral engaging edge configured to contact the capsular bag and a second coupling surface. The first and second coupling surfaces are in sliding contact with one another to permit movement of the power changing lens relative to the primary lens assembly and also to maintain a spaced relationship between the fixed lens and the lens cavity during radial compression of the power changing lens.

### SUMMARY

This disclosure provides an intraocular pseudophakic contact lens with a mechanism for securing by an anterior leaflet of a capsular wall and a related system and method.

According to the invention, an apparatus including an intraocular pseudophakic contact lens is provided in accordance with claim 1. Some optional or preferred features are set out in dependent claims.

Other technical features may be readily apparent to one skilled in the art from the following figures, descriptions, and claims.

### BRIEF DESCRIPTION OF THE DRAWINGS

For a more complete understanding of this disclosure and its features, reference is now made to the following description, taken in conjunction with the accompanying drawings, in which:
FIGURES 1 through 3 illustrate a first example intraocular pseudophakic contact lens according to this disclosure;
FIGURES 4 and 5 illustrate a second example intraocular pseudophakic contact lens according to this disclosure;
FIGURES 6 through 8 illustrate a third example intraocular pseudophakic contact lens according to this disclosure;
FIGURES 9 and 10 illustrate a fourth example intraocular pseudophakic contact lens according to this disclosure;
FIGURES 11 and 12 illustrate a fifth example intraocular pseudophakic contact lens according to this disclosure;
FIGURE 13 illustrates a sixth example intraocular pseudophakic contact lens according to this disclosure;
FIGURES 14 and 15 illustrate a seventh example intraocular pseudophakic contact lens according to this disclosure;
FIGURES 16 through 18 illustrate an eighth example intraocular pseudophakic contact lens according to this disclosure;
FIGURES 19 through 21 illustrate a ninth example intraocular pseudophakic contact lens according to this disclosure;
FIGURE 22 illustrates an example intraocular lens and an example intraocular pseudophakic contact lens in a patient's eye according to this disclosure; and
FIGURE 23 illustrates an example method for using an intraocular pseudophakic contact lens with an intraocular lens according to this disclosure.

### DETAILED DESCRIPTION

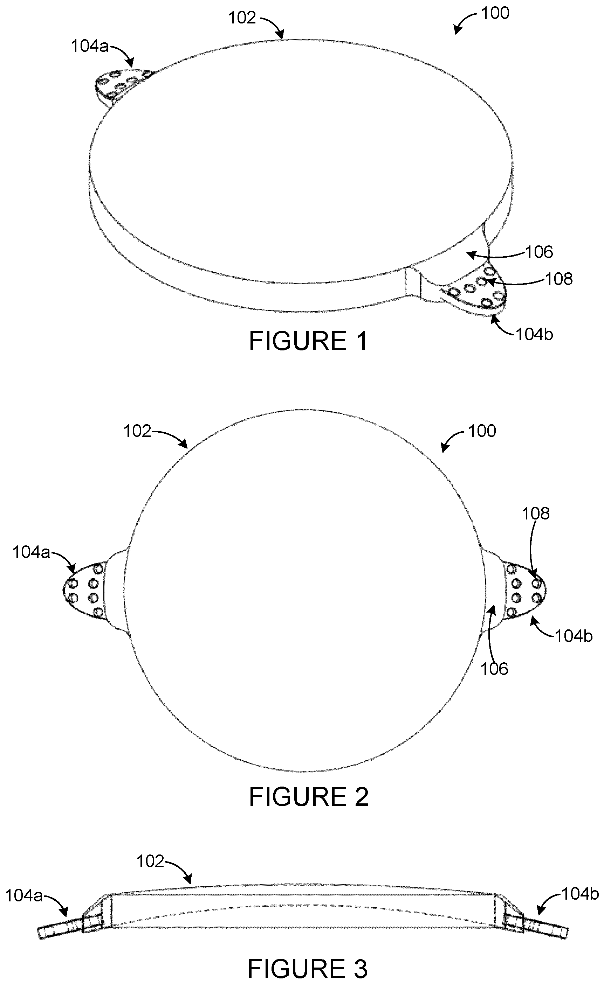

FIGURES 1 through 23, discussed below, and the various embodiments used to describe the principles of the present invention in this patent document are by way of illustration only and should not be construed in any way to limit the scope of the invention.

This disclosure provides various intraocular pseudophakic contact lenses (IOPCLs) that can be used in conjunction with intraocular lenses (IOLs). An intraocular pseudophakic contact lens generally represents a contact lens-type device that can be implanted within a patient's eye and placed on the anterior surface of an intraocular lens in the patient's eye. The intraocular pseudophakic contact lens substantially corrects residual refractive error present after implantation of the intraocular lens, such as after a lensectomy (cataract) procedure. In addition, the intraocular pseudophakic contact lens includes haptics or other mechanisms allowing the intraocular pseudophakic contact lens to be confined/captured by the anterior leaflet of the capsular wall in the patient's eye. In some instances, the haptics or other mechanisms can actually attach to the anterior leaflet of the capsular wall, such as through fibrosis during the healing process, to help to secure the intraocular pseudophakic contact lens in place.

Unlike conventional approaches, an intraocular pseudophakic contact lens can be implanted with less surgical risk. Moreover, an intraocular pseudophakic contact lens allows a patient to see immediately after implantation of the intraocular pseudophakic contact lens. Further, an intraocular pseudophakic contact lens can be easily replaced if a different lens is needed to correct residual refractive error or even removed if necessary. In addition, with techniques such as intraoperative wavefront aberrometry now available, refractive outcome can be measured during the actual procedure in which an intraocular pseudophakic contact lens is being implanted, which helps to identify immediately that a desired refractive target is obtained.

FIGURES 1 through 3 illustrate a first example intraocular pseudophakic contact lens 100 according to this disclosure. In particular, FIGURE 1 illustrates an oblique view of the intraocular pseudophakic contact lens 100, FIGURE 2 illustrates a top view of the intraocular pseudophakic contact lens 100, and FIGURE 3 illustrates a side view of the intraocular pseudophakic contact lens 100.

As shown in FIGURES 1 through 3, the intraocular pseudophakic contact lens 100 includes an optical lens 102. The optical lens 102 denotes the portion of the intraocular pseudophakic contact lens 100 that alters light passing through the intraocular pseudophakic contact lens 100. The light that passes through the optical lens 102 then travels through an associated intraocular lens before reaching the retina of a patient's eye.

The optical lens 102 can be formed from any suitable material(s), such as silicone or acrylic. The optical lens 102 can also be formed in any suitable manner, such as by using a mold or lathe cut manufacturing process. Different lenses 102 can be designed and manufactured to provide a wide range of diopters, and each optical lens 102 can be designed to correct any suitable refractive error(s). Example types of refractive errors that can be corrected include myopia, hyperopia, and astigmatism.

The optical lens 102 in this example has a convex top surface and a concave bottom surface. However, the optical lens 102 can have any other suitable shape, which could depend (at least in part) on the type of refractive error(s) being corrected. As particular examples, the optical lens 102 could be convex, concave, spherical, aspherical, toric, mono-focal, or multi-focal. The specific lens platform used as the optical lens 102 in the intraocular pseudophakic contact lens 100 can be selected to provide the desired refractive correction in a patient's eye. The optical lens 102 could also include various other features as needed or desired, such as when the optical lens 102 is weighted (like at its bottom) so that the optical lens 102 orients itself on an intraocular lens in a desired orientation (like for toric platforms) or when the optical lens 102 is tinted, is photochromic, or includes an ultraviolet (UV) absorber.

Multiple haptics 104a-104b extend from multiple sides of the optical lens 102. The haptics 104a-104b are sized and shaped so that they extend a short distance from the optical lens 102 and fit under the anterior leaflet of the capsular wall in a patient's eye after implantation. Each haptic 104a-104b could be formed from any suitable material(s) and in any suitable manner. For example, each haptic 104a-104b could be formed from the same material(s) as the optical lens 102. Note that while two haptics 104a-104b are shown here, the intraocular pseudophakic contact lens 100 could include any number of haptics, including a single haptic. Also note that while the haptics 104a-104b angle downward, the haptics 104a-104b could have any other suitable arrangement.

In this example, the haptics 104a-104b are separated from the optical lens 102 by projections or extensions 106 that project from the sides of the optical lens 102. These extensions 106 represents portions of the intraocular pseudophakic contact lens 100 in which ends of the haptics 104a-104b could be embedded. Each extension 106 could be formed from any suitable material(s) and in any suitable manner. For example, each extension 106 could represent a portion of the material(s) forming the optical lens 102 and therefore represent an extension of the optical lens 102 itself. However, this need not be the case. For instance, the optical lens 102 could be placed within a retaining ring that is integral with or attached to the extensions 106, or the extensions 106 could be secured to the optical lens 102 itself using adhesive or other suitable connecting mechanism.

Note that while two extensions 106 are shown here, the intraocular pseudophakic contact lens 100 could include any number of extensions, including a single extension. Also note that the presence of the extensions 106 is not required and that the haptics 104a-104b could be integrated directly with the optical lens 102. In those embodiments, the haptics 104a-104b could represent portions of the material(s) forming the optical lens 102, although this need not be the case. For instance, the optical lens 102 could be placed within a retaining ring that is integral with or attached to the haptics 104a-104b, or the haptics 104a-104b could be secured to the optical lens 102 itself using adhesive or other suitable connecting mechanism.

Each of the haptics 104a-104b includes a textured surface 108, which in this example is formed using various holes formed partially or completely through the haptics 104a-104b. The textured surfaces 108 allow the haptics 104a-104b to be captured and confined by the anterior leaflet of the capsular wall in a patient's pseudophakic eye. In some cases, the textured surfaces 108 allow the haptics 104a-104b to actually physically bond to the anterior leaflet of the capsular wall in the patient's eye, such as through fibrosis during the healing process. The haptics 104a-104b help to secure the intraocular pseudophakic contact lens 100 in place on an intraocular lens. Note that the numbers and sizes of the holes in the textured surfaces 108 are for illustration only and that the haptics 104a-104b could include different numbers and sizes of holes. For instance, the haptics 104a-104b could include a large number of very small holes or other structures forming a texture that promotes confinement, capture, or attachment to the anterior leaflet of the capsular wall.

The anterior leaflet of the capsular wall in a patient's eye is typically created during a capsulotomy in which the natural crystalline lens in the patient's eye is removed and replaced with an intraocular lens. The anterior leaflet represents the outer portion of the front side of the capsular bag that remains after an opening (referred to as a capsulorhexis) is formed in the capsular bag so that the natural crystalline lens can be removed. In some cases, this could occur long before the intraocular pseudophakic contact lens 100 is to be implanted. After the capsulotomy, the anterior leaflet of the capsular wall typically shrinks and undergoes fibrosis during the healing process.

When the intraocular pseudophakic contact lens 100 is inserted into the patient's eye, the intraocular pseudophakic contact lens 100 can be positioned so that the haptics 104a-104b extend under the anterior leaflet in the patient's eye. This allows the haptics 104a-104b to be captured and confined by the anterior leaflet. The haptics 104a-104b could also be physically attached to the anterior leaflet over time, such as by way of a "re-fibrosis" of the anterior leaflet. This re-fibrosis of tissue will bond to and cover part or all of the haptics 104a-104b, further securing the intraocular pseudophakic contact lens 100 in place. Note, however, that the intraocular pseudophakic contact lens 100 could also be implanted during the same procedure in which the intraocular lens is being implanted. In that case, the intraocular pseudophakic contact lens 100 could be secured by the haptics 104a-104b and possibly during fibrosis (and not re-fibrosis) within the patient's eye.

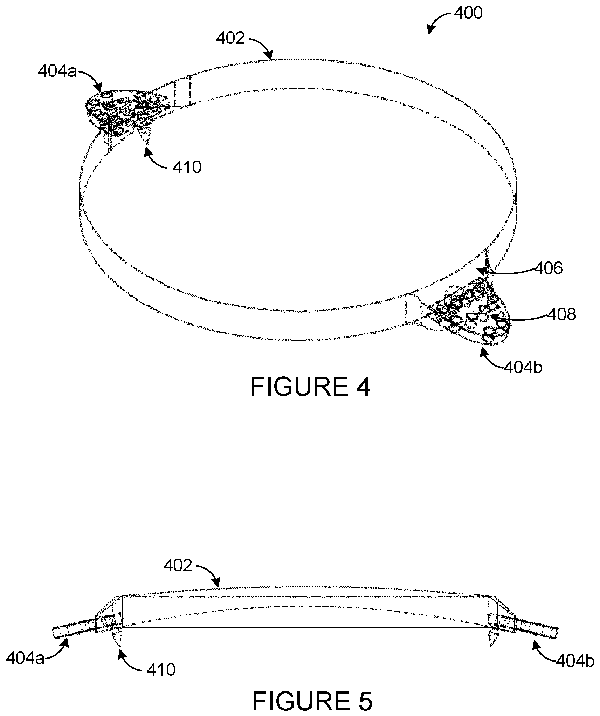

FIGURES 4 and 5 illustrate a second example intraocular pseudophakic contact lens 400 according to this disclosure. In particular, FIGURE 4 illustrates an oblique view of the intraocular pseudophakic contact lens 400, and FIGURE 5 illustrates a side view of the intraocular pseudophakic contact lens 400.

As shown in FIGURES 4 and 5, the intraocular pseudophakic contact lens 400 has various components that are the same as or similar to those forming the intraocular pseudophakic contact lens 100. For example, the intraocular pseudophakic contact lens 400 includes an optical lens 402, multiple haptics 404a-404b, and optionally multiple extensions 406. The haptics 404a-404b include textured surfaces 408.

The intraocular pseudophakic contact lens 400 also includes one or more pins 410. Each pin 410 projects downward from an extension 406 or from the inner end of a haptic 404a-404b. The pin(s) 410 can be used to pierce the anterior surface of an intraocular lens or to rest on the anterior surface of the intraocular lens. In addition to the capture/confinement of the haptics 404a-404b by the anterior leaflet, the pins 410 can help to further hold the intraocular pseudophakic contact lens 400 in place and resist slipping of the intraocular pseudophakic contact lens 400. In some cases, the pins 410 could be used to prevent movement of the intraocular pseudophakic contact lens 400 during the period immediately after implantation and before the haptics 404a-404b of the intraocular pseudophakic contact lens 400 have bonded to the anterior leaflet of the capsular wall in the patient's eye (such as via fibrosis).

Each pin 410 could be formed from any suitable material(s) and in any suitable manner. Note that while two pins 410 are shown here, the intraocular pseudophakic contact lens 400 could include any number of pins, including a single pin. Also note that while the pins 410 here are shown as having sharp ends, this need not be the case. For example, the pins 410 could have rounded or blunted surfaces to help the pins 410 sit on (without piercing) the anterior surface of an intraocular lens. In addition, while the pins 410 are shown here as extending through or being embedded within the extensions 406, the pins 410 could be located in any other suitable position(s). For instance, the pins 410 could be moved to the outer ends of the haptics 404a-404b, or additional pins 410 could be placed at the outer ends of the haptics 404a-404b.

As noted above, the intraocular pseudophakic contact lens 400 can be implanted during the same procedure in which an intraocular lens is being implanted or during a subsequent procedure after the intraocular lens has already been implanted in a patient's eye. The anterior leaflet of the capsular wall of the patient's eye could be used to capture and confine the haptics 404a-404b of the intraocular pseudophakic contact lens 400 under the anterior leaflet, and optionally fibrosis or re-fibrosis could occur to attach the haptics 404a-404b to the patient's eye. The pins 410 can be used to help hold the intraocular pseudophakic contact lens 400 in place.

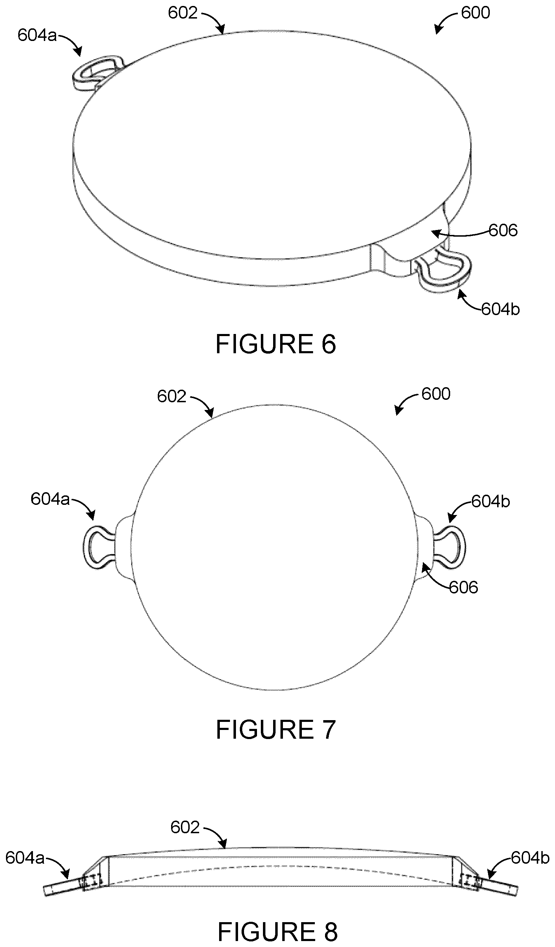

FIGURES 6 through 8 illustrate a third example intraocular pseudophakic contact lens 600 according to this disclosure. In particular, FIGURE 6 illustrates an oblique view of the intraocular pseudophakic contact lens 600, FIGURE 7 illustrates a top view of the intraocular pseudophakic contact lens 600, and FIGURE 8 illustrates a side view of the intraocular pseudophakic contact lens 600.

As shown in FIGURES 6 through 8, the intraocular pseudophakic contact lens 600 includes an optical lens 602, which may be the same as or similar to the optical lenses 202 and 402 described above. The intraocular pseudophakic contact lens 600 also includes multiple haptics 604a-604b and optionally multiple extensions 606. The haptics 604a-604b here are formed by loops of material, such as metal or plastic. The ends of the haptics 604a-604b are embedded within the extensions 606 in this example, although the extensions 606 could be omitted and the haptics 604a-604b could be coupled to the optical lens 602 or to a retaining ring in which the optical lens 602 is placed. Note that while the haptics 604a-604b angle downward, the haptics 604a-604b could have any other suitable arrangement. Each of the haptics 604a-604b could include a textured surface that facilitates confinement, capture, or attachment to the anterior leaflet of the capsular wall.

The intraocular pseudophakic contact lens 600 can be implanted during the same procedure in which an intraocular lens is being implanted or during a subsequent procedure after the intraocular lens has already been implanted in a patient's eye. The anterior leaflet of the capsular wall of the patient's eye could be used to capture and confine the haptics 604a-604b of the intraocular pseudophakic contact lens 600 under the anterior leaflet, and optionally fibrosis or re-fibrosis could occur to attach the haptics 604a-604b to the patient's eye.

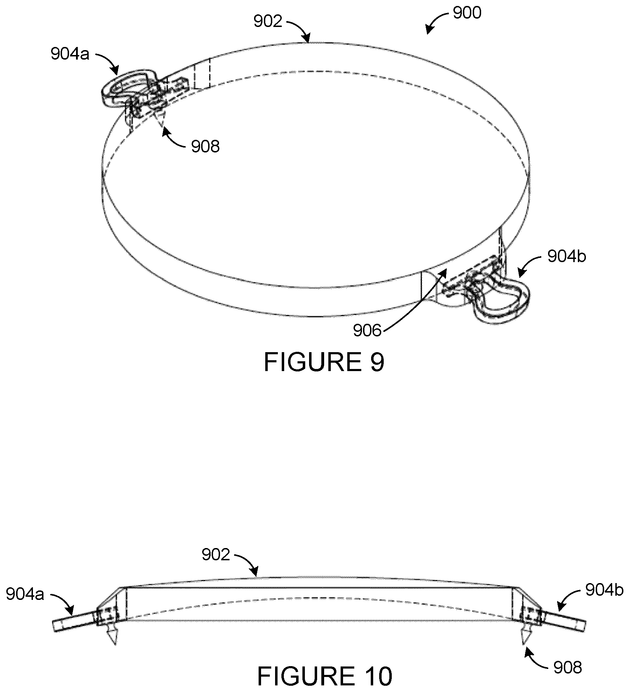

FIGURES 9 and 10 illustrate a fourth example intraocular pseudophakic contact lens 900 according to this disclosure. In particular, FIGURE 9 illustrates an oblique view of the intraocular pseudophakic contact lens 900, and FIGURE 10 illustrates a side view of the intraocular pseudophakic contact lens 900.

As shown in FIGURES 9 and 10, the intraocular pseudophakic contact lens 900 has various components that are the same as or similar to those forming the intraocular pseudophakic contact lens 600. For example, the intraocular pseudophakic contact lens 900 includes an optical lens 902, multiple haptics 904a-904b, and optionally multiple extensions 906. The haptics 904a-904b form small loops that are sized and shaped so that they extend a short distance from the optical lens 902 and fit under the anterior leaflet of the capsular wall in a patient's eye after implantation. Each of the haptics 904a-904b could include a textured surface that facilitates confinement, capture, or attachment to the anterior leaflet of the capsular wall.

The intraocular pseudophakic contact lens 900 also includes one or more pins 908. Each pin 908 projects downward from an extension 906 or from the inner end of a haptic 904a-904b. The pin(s) 908 can be used to pierce the anterior surface of an intraocular lens or to rest on the anterior surface of the intraocular lens. In addition to the capture/confinement of the haptics 904a-904b by the anterior leaflet, the pins 908 can help to further hold the intraocular pseudophakic contact lens 900 in place and resist slipping of the intraocular pseudophakic contact lens 900. In some cases, the pins 908 could be used to prevent movement of the intraocular pseudophakic contact lens 900 during the period immediately after implantation and before the haptics 904a-904b of the intraocular pseudophakic contact lens 900 have bonded to the anterior leaflet of the capsular wall in the patient's eye (such as via fibrosis).

Each pin 908 could be formed from any suitable material(s) and in any suitable manner. Note that while two pins 908 are shown here, the intraocular pseudophakic contact lens 900 could include any number of pins, including a single pin. Also note that while the pins 908 here are shown as having sharp ends, this need not be the case. For example, the pins 908 could have rounded or blunted surfaces to help the pins 908 sit on (without piercing) the anterior surface of an intraocular lens. In addition, while the pins 908 are shown here as extending through or being embedded within the extensions 906, the pins 908 could be located in any other suitable position(s). For instance, the pins 908 could be moved to the outer ends of the haptics 904a-904b, or additional pins 908 could be placed at the outer ends of the haptics 904a-904b.

The intraocular pseudophakic contact lens 900 can be implanted during the same procedure in which an intraocular lens is being implanted or during a subsequent procedure after the intraocular lens has already been implanted in a patient's eye. The anterior leaflet of the capsular wall of the patient's eye could be used to capture and confine the haptics 904a-904b of the intraocular pseudophakic contact lens 900 under the anterior leaflet, and optionally fibrosis or re-fibrosis could occur to attach the haptics 904a-904b to the patient's eye. The pins 908 can be used to help hold the intraocular pseudophakic contact lens 900 in place.

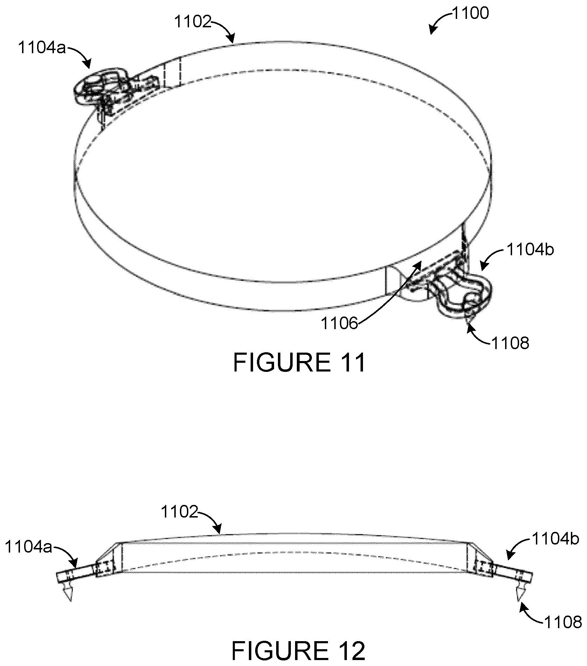

FIGURES 11 and 12 illustrate a fifth example intraocular pseudophakic contact lens 1100 according to this disclosure. In particular, FIGURE 11 illustrates an oblique view of the intraocular pseudophakic contact lens 1100, and FIGURE 12 illustrates a side view of the intraocular pseudophakic contact lens 1100.

As shown in FIGURES 11 and 12, the intraocular pseudophakic contact lens 1100 has various components that are the same as or similar to those forming the intraocular pseudophakic contact lens 900. For example, the intraocular pseudophakic contact lens 1100 includes an optical lens 1102, multiple haptics 1104a-1104b, and optionally multiple extensions 1106. The haptics 1104a-1104b form small loops that are sized and shaped so that they extend a short distance from the optical lens 1102 and fit under the anterior leaflet of the capsular wall in a patient's eye after implantation. Each of the haptics 1104a-1104b could include a textured surface that facilitates confinement, capture, or attachment to the anterior leaflet of the capsular wall.

The intraocular pseudophakic contact lens 1100 also includes one or more pins 1108. Each pin 1108 projects downward from the outer end of a haptic 1104a-1104b. The pins 1108 are therefore positioned farther away from the optical lens 1102 compared to the pins 908. This may allow the intraocular pseudophakic contact lens 1100 to be used with larger intraocular lenses. This may also allow the pins 1108 to extend to or beyond the edges of an intraocular lens, which could help the intraocular pseudophakic contact lens 1100 to lock onto the anterior surface or sides of the intraocular lens or to reduce sliding of the intraocular pseudophakic contact lens 1100 on the intraocular lens.

The intraocular pseudophakic contact lens 1100 can be implanted during the same procedure in which an intraocular lens is being implanted or during a subsequent procedure after the intraocular lens has already been implanted in a patient's eye. The anterior leaflet of the capsular wall of the patient's eye could be used to capture and confine the haptics 1104a-1104b of the intraocular pseudophakic contact lens 1100 under the anterior leaflet, and optionally fibrosis or re-fibrosis could occur to attach the haptics 1104a-1104b to the patient's eye. The pins 1108 can be used to help hold the intraocular pseudophakic contact lens 1100 in place.

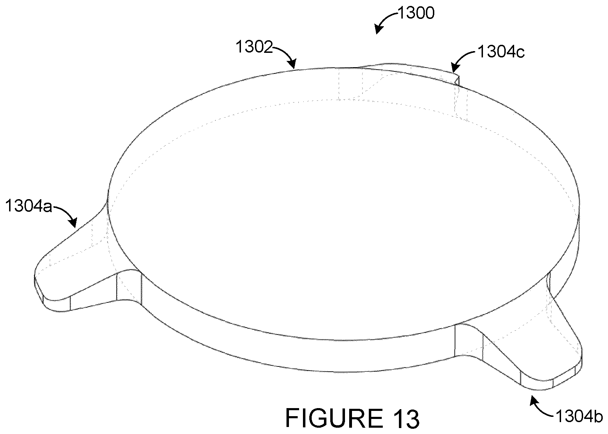

FIGURE 13 illustrates a sixth example intraocular pseudophakic contact lens 1300 according to this disclosure. As shown in FIGURE 13, the intraocular pseudophakic contact lens 1300 includes an optical lens 1302 and multiple haptics 1304a-1304c. The optical lens 1302 could be the same as or similar to the various optical lenses described above.

In this example, the haptics 1304a-1304c are formed by large projections that extend from the sides of the optical lens 1302, where the projections have a thickness that tapers towards the outer edges of the projections. This facilitates easier insertion of the haptics 1304a-1304c under the anterior leaflet of the capsular wall in a patient's eye. Each of the haptics 1304a-1304c could include a textured surface, such as a number of holes or other structures, that promotes confinement, capture, or attachment to the anterior leaflet of the capsular wall. While three haptics 1304a-1304c are shown here, other numbers of haptics could also be used.

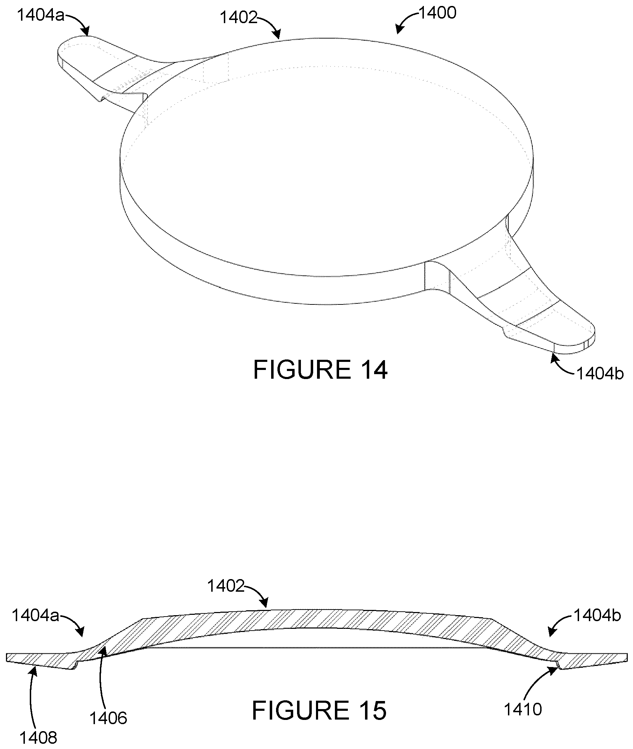

FIGURES 14 and 15 illustrate a seventh example intraocular pseudophakic contact lens 1400 according to this disclosure. In particular, FIGURE 14 illustrates an oblique view of the intraocular pseudophakic contact lens 1400, and FIGURE 15 illustrates a cross-sectional view through the middle of the intraocular pseudophakic contact lens 1400.

As shown in FIGURES 14 and 15, the intraocular pseudophakic contact lens 1400 includes an optical lens 1402 and multiple haptics 1404a-1404b. The optical lens 1402 could be the same as or similar to the various optical lenses described above. Each of the haptics 1404a-1404b include a textured surface, such as a number of holes or other structures, that promotes confinement, capture, or attachment to the anterior leaflet of the capsular wall.

In this example, the haptics 1404a-1404b are formed by larger projections that extend from the sides of the optical lens 1402. Each haptic 1404a-1404b includes an inner portion 1406 that is connected to the optical lens 1402 and an outer portion 1408 that is connected to the inner portion 1406, effectively forming long "wings" extending from the optical lens 1402. The outer portions 1408 have a thickness that tapers towards the outer edges of the haptics 1404a-1404b, which facilitates easier insertion of the haptics 1404a-1404b under the anterior leaflet of the capsular wall in a patient's eye. The inner portion 1406 projects outward and downward in this example, while the outer portion 1408 projects outward and slightly upward in this example (although other forms could also be used). This shape allows the haptics 1404a-1404b to be used with larger intraocular lenses while still extending under the anterior leaflet of the capsular wall.

Each of the haptics 1404a-1404b also includes a ridge 1410, and multiple ridges 1410 of multiple haptics 1404a-1404b can be used to capture one or more edges of the underlying intraocular lens. This can help to center the intraocular pseudophakic contact lens 1400 on the intraocular lens. This can also help to retain the intraocular pseudophakic contact lens 1400 in place on the intraocular lens during the healing process.

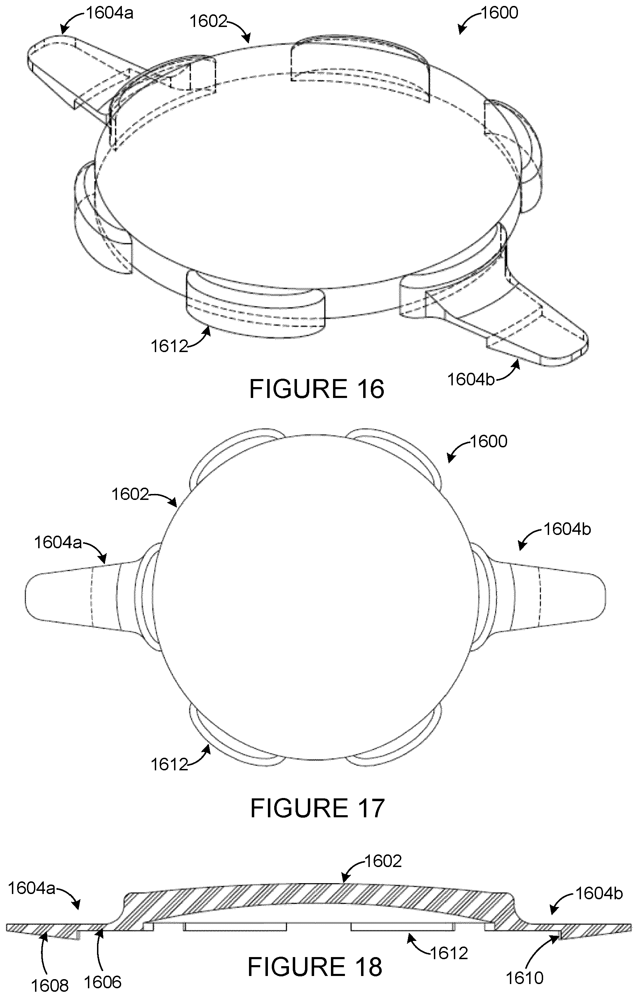

FIGURES 16 through 18 illustrate an eighth example intraocular pseudophakic contact lens 1600 according to this disclosure. In particular, FIGURE 16 illustrates an oblique view of the intraocular pseudophakic contact lens 1600, FIGURE 17 illustrates a top view of the intraocular pseudophakic contact lens 1600, and FIGURE 18 illustrates a cross-sectional view through the middle of the intraocular pseudophakic contact lens 1600.

As shown in FIGURES 16 through 18, the intraocular pseudophakic contact lens 1600 has various components that are the same as or similar to those forming the intraocular pseudophakic contact lens 1400. For example, the intraocular pseudophakic contact lens 1600 includes an optical lens 1602 and multiple haptics 1604a-1604b. The haptics 1604a-1604b are formed by larger projections that extend from the sides of the optical lens 1602. Each haptic 1604a-1604b includes an inner portion 1606 that is connected to the optical lens 1602 (or to a retaining ring in which the optical lens 1602 is located) and an outer portion 1608 that is connected to the inner portion 1606. The outer portions 1608 have a thickness that tapers towards the outer edges of the haptics 1604a-1604b, which facilitates easier insertion of the haptics 1604a-1604b under the anterior leaflet of the capsular wall in a patient's eye. The inner portion 1606 and the outer portion 1608 both project outward and straight in this example (although other forms could also be used). This shape allows the haptics 1604a-1604b to be used with larger intraocular lenses while still extending under the anterior leaflet of the capsular wall.

Each of the haptics 1604a-1604b also includes a ridge 1610, and multiple ridges 1610 of multiple haptics 1604a-1604b can be used to capture one or more edges of the underlying intraocular lens. This can help to center the intraocular pseudophakic contact lens 1600 on the intraocular lens. This can also help to retain the intraocular pseudophakic contact lens 1600 in place on the intraocular lens during the healing process.

In addition, the intraocular pseudophakic contact lens 1600 here includes multiple segments 1612 located along the sides of the optical lens 1602. The segments 1612 denote projections from the optical lens 1602, and at least some of the segments 1612 could be coupled to the haptics 1604a-1604b (such as when ends of the haptics 1604a-1604b are embedded in the segments 1612). The segments 1612 extend downward so that the bottom surfaces of the segments 1612 are located below the optical lens 1602. As a result, when implanted into a patient's eye, the segments 1612 keep the optical lens 1602 separated from the underlying intraocular lens. Depending on the shape of the posterior surface of the optical lens 1602 and the shape of the anterior surface of the underlying intraocular lens, this could elevate the optical lens 1602 over an optical lens within the underlying intraocular lens so that the optical lenses do not contact each other.

Each of the segments 1612 could be formed from any suitable material(s) and in any suitable manner. For example, each segment 1612 could represent a portion of the material(s) forming the optical lens 1602 and therefore represent an extension of the optical lens 1602 itself. However, this need not be the case. For instance, the optical lens 1602 could be placed within a retaining ring that is integral with or attached to the segments 1612, or the segments 1612 could be secured to the optical lens 1602 itself using adhesive or other suitable connecting mechanism. Each of the segments 1612 could also have any suitable size, shape, and dimensions. For example, the segments 1612 could be smaller or larger (relative to the other structures) than what is shown in FIGURES 16 through 18. As another example, the segments 1612 could denote curved structures that leave small open areas between the segments 1612 and the optical lens 1602, or the segments 1612 could be solid structures that leave no open areas between the segments 1612 and the optical lens 1602.

The ability to space the optical lens 1602 away from the underlying intraocular lens could provide various benefits. For example, elevating the optical lens 1602 over the underlying intraocular lens may allow for an increase in aqueous flow between the anterior surface of the intraocular lens and the posterior surface of the optical lens 1602. An increased flow of aqueous between the lenses could help to reduce lens deposits on either or both of the lenses. Also, the presence of aqueous between the lenses can help to improve the optic or image quality of the combined lens system. In addition, by providing more space between the lenses, the intraocular pseudophakic contact lens 1600 could be used with a wider range of intraocular lenses that have varying anterior curvature surfaces, allowing the intraocular pseudophakic contact lens 1600 to be used with a wider range of intraocular lens models and powers.

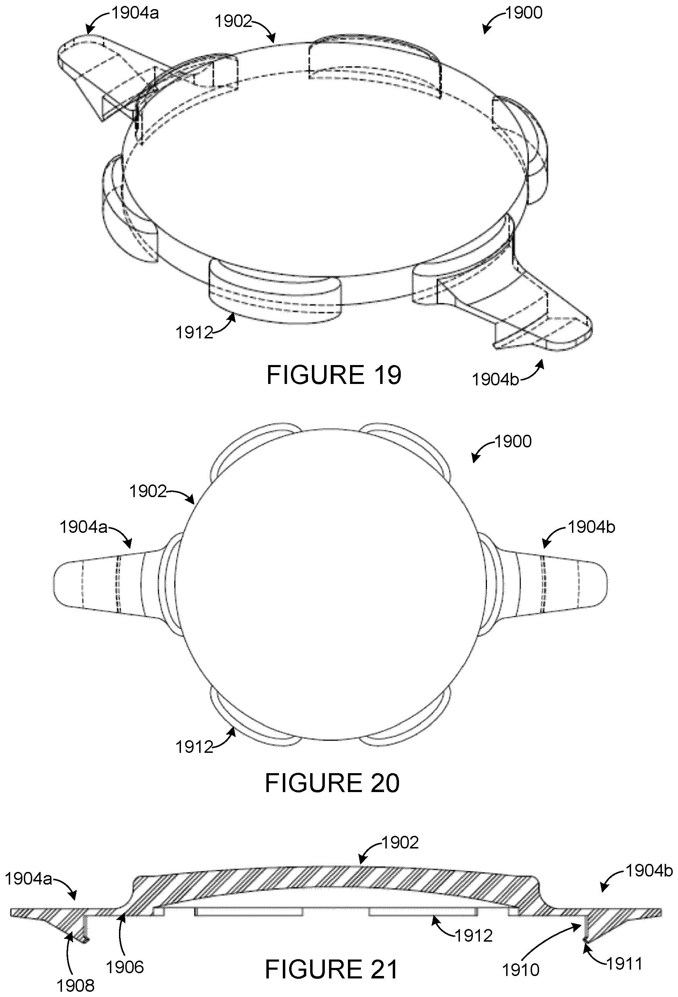

FIGURES 19 through 21 illustrate a ninth example intraocular pseudophakic contact lens 1900 according to this disclosure. In particular, FIGURE 19 illustrates an oblique view of the intraocular pseudophakic contact lens 1900, FIGURE 20 illustrates a top view of the intraocular pseudophakic contact lens 1900, and FIGURE 21 illustrates a cross-sectional view through the middle of the intraocular pseudophakic contact lens 1900.

As shown in FIGURES 19 through 21, the intraocular pseudophakic contact lens 1900 has various components that are the same as or similar to those forming the intraocular pseudophakic contact lens 1600. For example, the intraocular pseudophakic contact lens 1900 includes an optical lens 1902 and multiple haptics 1904a-1904b. The haptics 1904a-1904b are formed by larger projections that extend from the sides of the optical lens 1902. Each haptic 1904a-1904b includes an inner portion 1906 that is connected to the optical lens 1902 (or to a retaining ring in which the optical lens 1902 is located) and an outer portion 1908 that is connected to the inner portion 1906. Each of the haptics 1904a-1904b also includes a ridge 1910, and multiple ridges 1910 of multiple haptics 1904a-1904b can be used to capture one or more edges of the underlying intraocular lens. In addition, the intraocular pseudophakic contact lens 1900 includes multiple segments 1912 along the sides of the optical lens 1902. The segments 1912 extend downward so that the bottom surfaces of the segments 1912 are located below the optical lens 1902.

The haptics 1904a-1904b in this example include thicker outer portions 1908 with larger ridges 1910 compared to the corresponding components of the intraocular pseudophakic contact lens 1600. This allows the haptics 1904a-1904b to be used with even larger intraocular lenses. Moreover, each of the ridges 1910 includes a lip 1911 that can facilitate the capture of the underlying intraocular lens. Each of the lips 1911 denotes any suitable inward projection from the corresponding ridge 1910.

Again, when implanted into a patient's eye, the segments 1912 help to keep the optical lens 1902 separated from the underlying intraocular lens. Depending on the shape of the posterior surface of the optical lens 1902 and the shape of the anterior surface of the underlying intraocular lens, this could elevate the optical lens 1902 over an optical lens within the underlying intraocular lens so that the optical lenses do not contact each other. The ability to space the optical lens 1902 away from the underlying intraocular lens could provide various benefits, such as those described above with respect to the intraocular pseudophakic contact lens 1600.

While various prior approaches have secured an "add-on" lens to an intraocular lens, these prior approaches require a specific add-on lens to be designed for use with a specific intraocular lens and the specific intraocular lens to be designed for use with the specific add-on lens. That is, the add-on lens can only be used with a specific type of intraocular lens, where that intraocular lens is designed specifically for use with that add-on lens. As particular examples, an add-on lens may include haptics or other structures that are designed to mate with corresponding structures of specific intraocular lenses, or an intraocular lens may have a recess designed to receive a specific type of add-on lens. This can be problematic for a number of reasons. For instance, many patients already have existing intraocular lenses, and it may be impractical or even dangerous to try to remove those existing intraocular lenses in order to implant new intraocular lenses that are designed for use with add-on lenses.

The embodiments of the intraocular pseudophakic contact lenses shown in FIGURES 1 through 21 can help to alleviate these problems since the intraocular pseudophakic contact lenses can be secured over intraocular lenses by capturing and confining the haptics of the intraocular pseudophakic contact lenses using the anterior leaflets of capsular walls. In some cases, this could also involve physical bonding of the haptics to the anterior leaflets of capsular walls, such as via a fibrosis or re-fibrosis mechanism. In other words, the intraocular pseudophakic contact lenses do not need to be designed to work specifically with particular structures of any specific intraocular lens. The intraocular lens being used with an intraocular pseudophakic contact lens need not have any predefined structures that are provided for coupling to an intraocular pseudophakic contact lens. Rather, the intraocular pseudophakic contact lenses of FIGURES 1 through 21 can simply be sized so that, when the intraocular pseudophakic contact lens is placed on an intraocular lens, it can be secured in place through capture and confinement by (and possibly bonding with) the anterior leaflet of the capsular wall. This allows the intraocular pseudophakic contact lenses of FIGURES 1 through 21 to be used with a wide variety of intraocular lenses, including different types of intraocular lenses and including existing intraocular lenses already implanted into patients. There is no need to remove an existing intraocular lens from a patient in order to install a new intraocular lens and an intraocular pseudophakic contact lens.

Moreover, the intraocular pseudophakic contact lenses of FIGURES 1 through 21 could be easily removed from patients' eyes, such as any suitable time after implantation or prior to bonding of the haptics to the capsular walls (assuming fibrosis or re-fibrosis holds the intraocular pseudophakic contact lenses in place). Among other things, this allows one intraocular pseudophakic contact lens to be removed and replaced with a different intraocular pseudophakic contact lens if a different refractive correction is needed or desired.

The various intraocular pseudophakic contact lenses described above could have any suitable size, shape, and dimensions. For example, the intraocular pseudophakic contact lenses could be made available in a range of diameters from about 4mm to about 6mm. Also, the intraocular pseudophakic contact lenses could be made available with varying base curvatures for their optical lenses. Of course, an intraocular pseudophakic contact lens could also be custom designed for a particular patient's eye, such as when one or more specific curvatures are needed to correct for residual refractive error in the particular patient's eye.

The intraocular pseudophakic contact lenses disclosed here can be implanted non-invasively in patients' eyes and easily positioned on intraocular lenses. The implantation is non-invasive because an intraocular pseudophakic contact lens is being installed on the anterior surface of an intraocular lens, which is typically easily accessible by a surgeon or other personnel during an implantation procedure. The implantation is also non-invasive because the intraocular pseudophakic contact lenses can be attached to intraocular lenses without requiring attachment of the intraocular pseudophakic contact lenses to anatomical structures within the patients' eyes, such as to the suculus of a patient's eye.

The non-invasive implantation and easy positioning of an intraocular pseudophakic contact lens provide a safe and effective refractive surgical procedure to correct unwanted residual refractive error, such as after a lensectomy procedure. As a refractive modality, the intraocular pseudophakic contact lenses contribute to a surgeon's ability to alter the current refractive error of a pseudophakic patient in an effort to adjust the patient's vision to achieve a finely-tuned desired refraction. Specific examples of this functionality include allowing adjustments to a patient's eye in order to achieve unilateral or bilateral emmetropia, to induce unilateral myopia to allow for intermediate and near visual function, to introduce multi-focality, and to treat unwanted residual astigmatism.

If the haptics of an intraocular pseudophakic contact lens include ridges along their bottom surfaces, the ridges can be used to center the intraocular pseudophakic contact lens on an underlying intraocular lens as described above. If the intraocular pseudophakic contact lens includes three haptics with associated ridges, the ridges could help to perfectly center the intraocular pseudophakic contact lens on the underlying intraocular lens. Such an approach allows the ridges of the intraocular pseudophakic contact lens' haptics to capture the underlying intraocular lens at the edge and perfectly line up the optical center of the intraocular pseudophakic contact lens' optic with the optical center of the intraocular lens. This alignment helps to reduce or avoid induced optical aberrations or induced prisms caused by optical center misalignment. This provides a strong contributing benefit over conventional refractive fine-tuning modalities.

Note that in any of the above examples, the intraocular pseudophakic contact lens could possibly be designed so that only the haptics of the intraocular pseudophakic contact lens extend under the anterior leaflet of the capsular wall in a patient's eye. This allows the haptics to be captured and confined by the anterior leaflet while leaving the optical lens of the intraocular pseudophakic contact lens free and generally unobscured by the surrounding tissue in the patient's eye.

Although FIGURES 1 through 21 illustrate examples of intraocular pseudophakic contact lens, various changes may be made to FIGURES 1 through 21. For example, any suitable combination of features shown in FIGURES 1 through 21 could be used together in a single intraocular pseudophakic contact lens, whether or not that specific combination of features is shown in the figures or described above. As a particular example, any of the intraocular pseudophakic contact lenses shown in FIGURES 1 through 21 could include one or more pins at one or more desired locations, one or more ridges along the bottom surface(s) of its haptic(s) to aid in the capture of an intraocular lens and centration on the intraocular lens, and/or one or more lips to help capture the intraocular lens. Also, each intraocular pseudophakic contact lens could include any suitable number of each component shown in any of the figures. While the figures have shown the intraocular pseudophakic contact lenses as having two or three haptics at an even spacing of 120° or 180°, any number of haptics (with or without associated pins, ridges, lips, or other structures) could be used. Further, the forms of the haptics shown here are examples only, and any other suitable structures could be used to capture, confine, or attach to the anterior leaflet of a capsular wall in a patient's eye. In addition, a number of other features could be used at one or more locations of the intraocular pseudophakic contact lenses. For instance, one or more alignment markings could be provided to identify proper alignment of the intraocular pseudophakic contact lens with the intraocular lens, or one or more drug-eluting materials could be placed on top, side, or bottom surfaces of the optical lenses in the intraocular pseudophakic contact lenses.

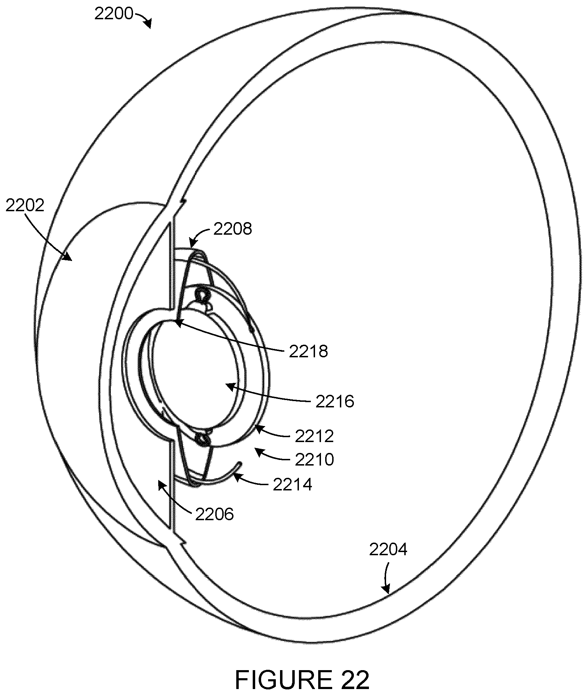

FIGURE 22 illustrates an example intraocular lens and an example intraocular pseudophakic contact lens in a patient's eye 2200 according to this disclosure. As shown in FIGURE 22, the eye 2200 includes a cornea 2202, a sclera 2204, and an iris 2206. The cornea 2202 represents the clear front portion of the eye 2200 through which light passes to enter into the eye 2200. The sclera 2204 is the tough outer white portion of the eye. The iris 2206 controls the size of the eye's pupil to thereby control the amount of light from the cornea 2202 that enters into the interior of the eye 2200.

The eye 2200 also includes a capsular bag 2208, which typically holds the natural crystalline lens of the eye 2200. However, in this example, the natural crystalline lens has been removed and replaced with an intraocular lens 2210 having an optical lens 2212 and one or more haptics 2214. The optical lens 2212 of the intraocular lens 2210 receives light entering the eye and focuses the light onto the retina of the eye 2200. The haptics 2214 of the intraocular lens 2210 help to hold the intraocular lens 2210 within the capsular bag 2208 so that the optical lens 2212 of the intraocular lens 2210 is in a desired position within the eye.

An intraocular pseudophakic contact lens 2216 has also been placed on the intraocular lens 2210 within the capsular bag 2208. The intraocular pseudophakic contact lens 2216 can represent any of the intraocular pseudophakic contact lenses described above or any other suitable intraocular pseudophakic contact lens. The intraocular pseudophakic contact lens 2216 is placed on the anterior surface of the intraocular lens 2210, meaning the front surface of the intraocular lens 2210 with respect to the eye 2200. Light enters through the cornea 2202 and passes through the pupil before entering the intraocular pseudophakic contact lens 2216, which modifies the light. The modified light then passes through the optical lens 2212 of the intraocular lens 2210 and is again modified. The twice-modified light then travels through the remainder of the eye 2200 to reach the retina at the back of the eye 2200.

As described above, the intraocular pseudophakic contact lens 2216 includes one or more haptics that extend a short distance and fit under an anterior leaflet 2218 of the capsular bag 2208. This allows the haptics to be captured and confined by the anterior leaflet 2218 (and possibly attach to the anterior leaflet 2218 via fibrosis or re-fibrosis). The anterior leaflet 2218 represents the outer portion of the front side of the capsular bag 2208 that remains after a capsulorhexis is formed in the capsular bag 2208. The insertion of the haptics of the intraocular pseudophakic contact lens 2216 under the anterior leaflet 2218 helps to secure the intraocular pseudophakic contact lens 2216 in place. In some cases, the healing process in the eye 2200 can cause fibrosis to occur, which could also attach the anterior leaflet 2218 to the haptics of the intraocular pseudophakic contact lens 2216.

Note that the haptics of the intraocular pseudophakic contact lens 2216 are shorter or smaller than the haptics 2214 of the intraocular lens 2210. This is because the haptics 2214 of the intraocular lens 2210 extend generally to the top and bottom of the capsular bag 2208 and help to hold the intraocular lens 2210 in the proper positon within the capsular bag 2208. The haptics of the intraocular pseudophakic contact lens 2216 need not extend to the top and bottom of the capsular bag 2208 and instead may only extend a short distance under the anterior leaflet 2218.

By properly selecting the optical lens of the intraocular pseudophakic contact lens 2216, the intraocular pseudophakic contact lens 2216 can ideally correct any residual refractive error that remains after implantation of the intraocular lens 2210. If necessary, the intraocular pseudophakic contact lens 2216 can also be removed and replaced with a different intraocular pseudophakic contact lens. This may be needed or desired if the intraocular pseudophakic contact lens 2216 does not properly correct the residual refractive error or if the intraocular pseudophakic contact lens 2216 actually causes additional refractive errors.

Although FIGURE 22 illustrates one example of an intraocular lens and one example of an intraocular pseudophakic contact lens in a patient's eye, various changes may be made to FIGURE 22. For example, the intraocular lens 2210 could be attached to any other intraocular pseudophakic contact lens. Also, there are a number of intraocular lenses available, and an intraocular pseudophakic contact lens could be coupled to any other suitable intraocular lens in the eye 2200.

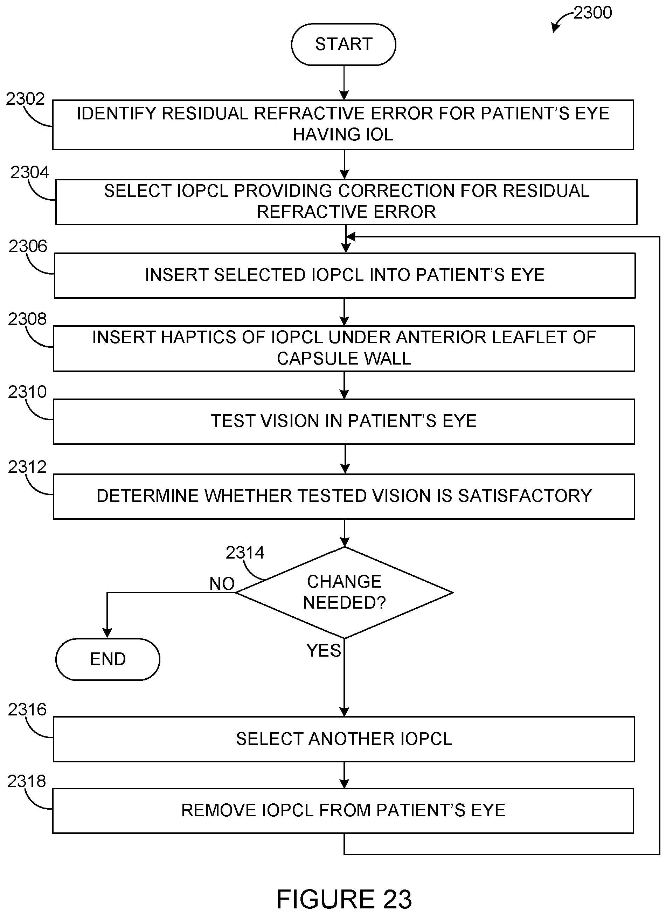

FIGURE 23 illustrates an example method 2300 for using an intraocular pseudophakic contact lens with an intraocular lens according to this disclosure. As shown in FIGURE 23, residual refractive error in a patient's eye having an intraocular lens is identified at step 2302. This could include, for example, personnel testing the patient's vision and identifying any refractive error that remains after implantation of the intraocular lens 2210. The testing could be done in any suitable manner, such as by using intraoperative wavefront aberrometry. One goal of the testing can be to identify what refractive errors exist in the patient's eye after implantation of the intraocular lens in the patient's eye. This testing could be performed at any suitable time, such as after a lensectomy procedure.

An intraocular pseudophakic contact lens (IOPCL) is selected to (ideally) correct the identified residual refractive error at step 2304. This could include, for example, personnel selecting an intraocular pseudophakic contact lens from a kit, where the selected intraocular pseudophakic contact lens has an optical lens that substantially neutralizes the identified residual refractive error. This could also include the personnel selecting an optical lens from a kit and inserting the optical lens into an intraocular pseudophakic contact lens, where the selected optical lens substantially cancels the identified residual refractive error. This could further include the personnel obtaining an intraocular pseudophakic contact lens having a custom-designed optical lens or obtaining a custom-designed optical lens for insertion into an intraocular pseudophakic contact lens, where the custom-designed optical lens substantially cancels the identified residual refractive error. In general, any mechanism can be used to obtain a suitable intraocular pseudophakic contact lens.

The selected intraocular pseudophakic contact lens is inserted into the patient's eye at step 2306. This could include, for example, a surgeon or other personnel forming a small incision in the patient's eye and inserting the intraocular pseudophakic contact lens into the eye through the incision. The intraocular pseudophakic contact lens can be rolled, folded, or otherwise reduced in cross-sectional size in order to insert the intraocular pseudophakic contact lens through a smaller incision.

One or more haptics of the intraocular pseudophakic contact lens are inserted under the anterior leaflet of the capsular wall in the patient's eye at step 2308. This could include, for example, the surgeon or other personnel placing the intraocular pseudophakic contact lens 2216 at a desired position (and possibly in a desired orientation) on the intraocular lens 2210. This could also include the surgeon or other personnel moving the intraocular pseudophakic contact lens 2216 so that its haptics (in whatever form) slide under the anterior leaflet 2218 of the capsular bag 2208.

A vision test for the patient occurs at step 2310. The vision test could be done in any suitable manner, such as by using intraoperative wavefront aberrometry. This vision test could also be performed at any suitable time, such as during the surgical procedure in which the intraocular pseudophakic contact lens is being implanted or after the surgical procedure has been completed. A determination is made whether the tested vision is satisfactory at step 2312. This could include, for example, personnel determining whether the patient's eye is still experiencing any residual refractive error and, if so, to what extent.

A determination is made whether to change the intraocular pseudophakic contact lens at step 2314. This could include, for example, the personnel and the patient determining whether the remaining residual refractive error (if any) is inconvenient or otherwise problematic for the patient. If so, different steps could be taken to try and fix the problem. For instance, the currently-implanted intraocular pseudophakic contact lens could be repositioned to adjust for cylinder axis correction. If that fails, another intraocular pseudophakic contact lens is selected at step 2316. This could include, for example, personnel selecting another intraocular pseudophakic contact lens that (ideally) provides a better refractive correction for the patient's eye compared to the currently-inserted intraocular pseudophakic contact lens. The currently-inserted intraocular pseudophakic contact lens is removed from the patient's eye at step 2318. This could include, for example, the surgeon or other personnel sliding the haptics of the currently-inserted intraocular pseudophakic contact lens 2216 from under the anterior leaflet 2218 and removing the currently-inserted intraocular pseudophakic contact lens 2216 from the patient's eye. The process then returns to step 2306, where the newly-selected intraocular pseudophakic contact lens can be inserted into the patient's eye and the vision test can be repeated.

After the process shown in FIGURE 23 is over, the haptics of the implanted intraocular pseudophakic contact lens 2216 can be captured/confined by the anterior leaflet 2218 of the capsular bag 2208 in the patient's eye. This helps to hold the intraocular pseudophakic contact lens 2216 in place. Moreover, the healing process in the patient's eye can optionally cause fibrosis or re-fibrosis to occur, which could physically attach the haptics of the implanted intraocular pseudophakic contact lens 2216 to the anterior leaflet 2218 of the capsular bag 2208.

Although FIGURE 23 illustrates one example of a method 2300 for using an intraocular pseudophakic contact lens with an intraocular lens, various changes may be made to FIGURE 23. For example, while shown as a series of steps, various steps in FIGURE 23 could overlap, occur in parallel, occur in a different order, or occur any number of times.

It may be advantageous to set forth definitions of certain words and phrases used throughout this patent document. The terms "include" and "comprise," as well as derivatives thereof, mean inclusion without limitation. The term "or" is inclusive, meaning and/or. The phrase "associated with," as well as derivatives thereof, may mean to include, be included within, interconnect with, contain, be contained within, connect to or with, couple to or with, be communicable with, cooperate with, interleave, juxtapose, be proximate to, be bound to or with, have, have a property of, have a relationship to or with, or the like. The phrase "at least one of," when used with a list of items, means that different combinations of one or more of the listed items may be used, and only one item in the list may be needed. For example, "at least one of: A, B, and C" includes any of the following combinations: A, B, C, A and B, A and C, B and C, and A and B and C.

## Claims

1. An apparatus comprising:
an intraocular pseudophakic contact lens (1400, 1600, 1900) comprising:
an optical lens (1402, 1602, 1902) configured to at least partially correct a residual refractive error in an eye, the residual refractive error comprising a refractive error that exists in the eye after implantation of an artificial intraocular lens (2210) in the eye; and
haptics (1404a-1404b, 1604a-1604b, 1904a-1904b) extending radially from the optical lens (1402, 1602, 1902) and configured to be inserted under an anterior leaflet of a capsular wall in the eye in order to capture and confine the haptics (1404a-1404b, 1604a-1604b, 1904a-1904b) under the anterior leaflet and secure the intraocular pseudophakic contact lens (1400, 1600, 1900) against the artificial intraocular lens (2210);
wherein anterior surfaces of the haptics (1404a-1404b, 1604a-1604b, 1904a-1904b) comprise textured capsular wall-engaging surfaces configured to contact an inner capsular wall surface of the anterior leaflet, the textured capsular wall-engaging surfaces configured to promote confinement, capture, or attachment of the haptics (1404a-1404b, 1604a-1604b, 1904a-1904b); and
wherein posterior surfaces of the haptics (1404a-1404b, 1604a-1604b, 1904a-1904b) comprise respective ridges (1410, 1610, 1910) configured to capture at least one edge of the artificial intraocular lens (2210) in order to secure the intraocular pseudophakic contact lens (1400, 1600, 1900) to the artificial intraocular lens (2210).

2. The apparatus of Claim 1, wherein:
each of the haptics (1404a-1404b, 1604a-1604b, 1904a-1904b) comprises an inner portion (1406, 1606, 1906) and an outer portion (1408, 1608, 1908);
the inner portion (1406, 1606, 1906) of each haptic (1404a-1404b, 1604a-1604b, 1904a-1904b) couples the optical lens (1402, 1602, 1902) and the outer portion (1408, 1608, 1908) of the haptic (1404a-1404b, 1604a-1604b, 1904a-1904b); and
the outer portion (1408, 1608, 1908) of each haptic (1404a-1404b, 1604a-1604b, 1904a-1904b) has a thickness that tapers from a larger thickness adjacent the inner portion (1406, 1606, 1906) of the haptic (1404a-1404b, 1604a-1604b, 1904a-1904b) to a smaller thickness adjacent an outer edge of the haptic (1404a-1404b, 1604a-1604b, 1904a-1904b).

3. The apparatus of Claim 2, wherein the inner portion (1406, 1606, 1906) of each haptic (1404a-1404b, 1604a-1604b, 1904a-1904b) projects outward and downward from the optical lens (1402, 1602, 1902).

4. The apparatus of Claim 3, wherein the outer portion (1408, 1608, 1908) of each haptic (1404a-1404b, 1604a-1604b, 1904a-1904b) projects outward and upward from the inner portion (1406, 1606, 1906) of the haptic (1404a-1404b, 1604a-1604b, 1904a-1904b).

5. The apparatus of any of Claims 1-4, wherein:
an anterior surface of the optical lens (1402, 1602, 1902) is convex; and
a posterior surface of the optical lens (1402, 1602, 1902) is concave.

6. The apparatus of any of Claims 1-5, wherein the intraocular pseudophakic contact lens (1400, 1600, 1900) comprises at least three haptics (1404a-1404b, 1604a-1604b, 1904a-1904b).

7. The apparatus of any of Claims 1-6, wherein the textured capsular wall-engaging surfaces are configured to bond to the inner capsular wall surface of the anterior leaflet through fibrosis.

8. The apparatus of any of Claims 1-7, wherein the intraocular pseudophakic contact lens (1400, 1600, 1900) further comprises one or more alignment markings to identify proper alignment of the intraocular pseudophakic contact lens (1400, 1600, 1900) with the artificial intraocular lens (2210).

9. The apparatus of any of Claims 1-8, wherein the optical lens (1402, 1602, 1902) is multi-focal.

10. A system comprising:
an artificial intraocular lens (2210) configured to be implanted within a capsular bag in an eye; and
the apparatus of any of Claims 1-9.

11. The system of Claim 10, wherein the haptics (1404a-1404b, 1604a-1604b, 1904a-1904b) of the intraocular pseudophakic contact lens (1400, 1600, 1900) are shorter than haptics (2214) of the artificial intraocular lens (2210).

## Patentansprüche

1. Vorrichtung, umfassend:
eine intraokulare pseudophakische Kontaktlinse (1400, 1600, 1900), umfassend:a
eine optische Linse (1402, 1602, 1902), die dazu konfiguriert ist, einen zurückgebliebenen Brechungsfehler in einem Auge wenigstens teilweise zu korrigieren, wobei der zurückgebliebene Brechungsfehler einen Brechungsfehler umfasst, der nach Implantation einer künstlichen intraokularen Linse (2210) in das Auge in dem Auge vorliegt; und
Haptiken (1404a-1404b, 1604a-1604b, 1904a-1904b), die sich von der optischen Linse (1402, 1602, 1902) radial erstrecken und dazu konfiguriert sind, unter eine anteriore Schicht einer Kapselwand in dem Auge eingefügt zu werden, um die Haptiken (1404a-1404b, 1604a-1604b, 1904a-1904b) unter der anterioren Schicht einzufassen und festzuhalten und die intraokulare pseudophakische Kontaktlinse (1400, 1600, 1900) gegen die künstliche intraokulare Linse (2210) zu befestigen;
wobei anteriore Flächen der Haptiken (1404a-1404b, 1604a-1604b, 1904a-1904b) texturierte Kapselwandeingriffsflächen umfassen, die dazu konfiguriert sind, eine innere Kapselwandfläche der anterioren Schicht zu kontaktieren, wobei die texturierten Kapselwandeingriffsflächen dazu konfiguriert sind, Festhalten, Einfassung oder Anbringung der Haptiken (1404a-1404b, 1604a-1604b, 1904a-1904b) zu fördern; und
wobei posteriore Flächen der Haptiken (1404a-1404b, 1604a-1604b, 1904a-1904b) jeweilige Erhöhungen (1410, 1610, 1910) umfassen, die dazu konfiguriert sind, mindestens einen Rand der künstlichen intraokularen Linse (2210) einzufassen, um die intraokulare pseudophakische Kontaktlinse (1400, 1600, 1900) an der künstlichen intraokularen Linse (2210) zu befestigen.

2. Vorrichtung nach Anspruch 1, wobei:
jede der Haptiken (1404a-1404b, 1604a-1604b, 1904a-1904b) einen inneren Abschnitt (1406, 1606, 1906) und einen äußeren Abschnitt (1408, 1608, 1908) umfasst;
der innere Abschnitt (1406, 1606, 1906) jeder Haptik (1404a-1404b, 1604a-1604b, 1904a-1904b) die optische Linse (1402, 1602, 1902) und den äußeren Abschnitt (1408, 1608, 1908) der Haptik (1404a-1404b, 1604a-1604b, 1904a-1904b) koppelt; und
der äußere Abschnitt (1408, 1608, 1908) jeder Haptik (1404a-1404b, 1604a-1604b, 1904a-1904b) eine Dicke aufweist, die sich von einer größeren Dicke angrenzend an den inneren Abschnitt (1406, 1606, 1906) der Haptik (1404a-1404b, 1604a-1604b, 1904a-1904b) zu einer geringeren Dicke angrenzend an einen äußeren Rand der Haptik (1404a-1404b, 1604a-1604b, 1904a-1904b) verjüngt.

3. Vorrichtung nach Anspruch 2, wobei der innere Abschnitt (1406, 1606, 1906) jeder Haptik (1404a-1404b, 1604a-1604b, 1904a-1904b) nach außen und nach unten von der optischen Linse (1402, 1602, 1902) hervorsteht.

4. Vorrichtung nach Anspruch 3, wobei der äußere Abschnitt (1408, 1608, 1908) jeder Haptik (1404a-1404b, 1604a-1604b, 1904a-1904b) nach außen und nach oben von dem inneren Abschnitt (1406, 1606, 1906) der Haptik (1404a-1404b, 1604a-1604b, 1904a-1904b) hervorsteht.

5. Vorrichtung nach einem der Ansprüche 1-4,
wobei: eine anteriore Fläche der optischen Linse (1402, 1602, 1902) konvex ist; und
eine posteriore Fläche der optischen Linse (1402, 1602, 1902) konkav ist.

6. Vorrichtung nach einem der Ansprüche 1-5, wobei die intraokulare pseudophakische Kontaktlinse (1400, 1600, 1900) mindestens drei Haptiken (1404a-1404b, 1604a-1604b, 1904a-1904b) umfasst.

7. Vorrichtung nach einem der Ansprüche 1-6, wobei die texturierten Kapselwandeingriffsflächen dazu konfiguriert sind, sich durch Fibrose mit der inneren Kapselwandfläche der anterioren Schicht zu verbinden.

8. Vorrichtung nach einem der Ansprüche 1-7, wobei die intraokulare pseudophakische Kontaktlinse (1400, 1600, 1900) ferner eine oder mehrere Ausrichtungsmarkierungen umfasst, um eine ordnungsgemäße Ausrichtung der intraokularen pseudophakischen Kontaktlinse (1400, 1600, 1900) mit der künstlichen intraokularen Linse (2210) zu identifizieren.

9. Vorrichtung nach einem der Ansprüche 1-8, wobei die optische Linse (1402, 1602, 1902) multifokal ist.

10. System, umfassend:
eine künstliche intraokulare Linse (2210), die dazu konfiguriert ist, innerhalb eines Kapselsacks in einem Auge implantiert zu sein; und
die Vorrichtung nach einem der Ansprüche 1-9.

11. System nach Anspruch 10, wobei die Haptiken (1404a-1404b, 1604a-1604b, 1904a-1904b) der intraokularen pseudophakischen Kontaktlinse (1400, 1600, 1900) kürzer als Haptiken (2214) der künstlichen intraokularen Linse (2210) sind.

## Revendications

1. Appareil comprenant :
une lentille de contact pseudophake intraoculaire (1400, 1600, 1900) comprenant :
une lentille optique (1402, 1602, 1902) configurée pour corriger au moins partiellement une erreur de réfraction résiduelle dans un œil, l'erreur de réfraction résiduelle comprenant une erreur de réfraction qui existe dans l'œil après l'implantation d'une lentille intraoculaire artificielle (2210) dans l'œil ; et
des haptiques (1404a-1404b, 1604a-1604b, 1904a-1904b) s'étendant radialement à partir de la lentille optique (1402, 1602, 1902) et configurées pour être insérées sous un feuillet antérieur d'une paroi capsulaire dans l'œil afin de capturer et de confiner les haptiques (1404a-1404b, 1604a-1604b, 1904a-1904b) sous le feuillet antérieur et de fixer la lentille de contact pseudophake intraoculaire (1400, 1600, 1900) contre la lentille intraoculaire artificielle (2210) ;
dans lequel des surfaces antérieures des haptiques (1404a-1404b, 1604a-1604b, 1904a-1904b) comprennent des surfaces texturées de mise en prise avec la paroi capsulaire configurées pour entrer en contact avec une surface de paroi capsulaire interne du feuillet antérieur, les surfaces texturées de mise en prise avec la paroi capsulaire étant configurées pour favoriser le confinement, la capture ou la fixation des haptiques (1404a-1404b, 1604a-1604b, 1904a-1904b) ; et
dans lequel des surfaces postérieures des haptiques (1404a-1404b, 1604a-1604b, 1904a-1904b) comprennent des nervures respectives (1410, 1610, 1910) configurées pour capturer au moins un bord de la lentille intraoculaire artificielle (2210) afin de fixer la lentille de contact pseudophake intraoculaire (1400, 1600, 1900) à la lentille intraoculaire artificielle (2210).

2. Appareil selon la revendication 1, dans lequel :
chacune des haptiques (1404a-1404b, 1604a-1604b, 1904a-1904b) comprend une partie interne (1406, 1606, 1906) et une partie externe (1408, 1608, 1908) ;
la partie interne (1406, 1606, 1906) de chaque haptique (1404a-1404b, 1604a-1604b, 1904a-1904b) couple la lentille optique (1402, 1602, 1902) et la partie externe (1408, 1608, 1908) de l'haptique (1404a-1404b, 1604a-1604b, 1904a-1904b) ; et
la partie externe (1408, 1608, 1908) de chaque haptique (1404a-1404b, 1604a-1604b, 1904a-1904b) présente une épaisseur qui s'amincit à partir d'une épaisseur plus grande adjacente à la partie interne (1406, 1606, 1906) de l'haptique (1404a-1404b, 1604a-1604b, 1904a-1904b) jusqu'à une épaisseur plus petite adjacente à un bord externe de l'haptique (1404a-1404b, 1604a-1604b, 1904a-1904b).

3. Appareil selon la revendication 2, dans lequel la partie interne (1406, 1606, 1906) de chaque haptique (1404a-1404b, 1604a-1604b, 1904a-1904b) fait saillie vers l'extérieur et vers le bas à partir de la lentille optique (1402, 1602, 1902).

4. Appareil selon la revendication 3, dans lequel la partie externe (1408, 1608, 1908) de chaque haptique (1404a-1404b, 1604a-1604b, 1904a-1904b) fait saillie vers l'extérieur et vers le haut à partir de la partie interne (1406, 1606, 1906) de l'haptique (1404a-1404b, 1604a-1604b, 1904a-1904b).

5. Appareil selon l'une quelconque des revendications 1 à 4,
dans lequel : une surface antérieure de la lentille optique (1402, 1602, 1902) est convexe ; et
une surface postérieure de la lentille optique (1402, 1602, 1902) est concave.

6. Appareil selon l'une quelconque des revendications 1 à 5, dans lequel la lentille de contact pseudophake intraoculaire (1400, 1600, 1900) comprend au moins trois haptiques (1404a-1404b, 1604a-1604b, 1904a-1904b).

7. Appareil selon l'une quelconque des revendications 1 à 6, dans lequel les surfaces texturées de mise en prise avec la paroi capsulaire sont configurées pour se lier à la surface de la paroi capsulaire interne du feuillet antérieur par fibrose.

8. Appareil selon l'une quelconque des revendications 1 à 7, dans lequel la lentille de contact pseudophake intraoculaire (1400, 1600, 1900) comprend en outre une ou plusieurs marques d'alignement pour identifier un alignement correct de la lentille de contact pseudophake intraoculaire (1400, 1600, 1900) avec la lentille intraoculaire artificielle (2210).

9. Appareil selon l'une quelconque des revendications 1 à 8, dans lequel la lentille optique (1402, 1602, 1902) est multifocale.

10. Système comprenant :
une lentille intraoculaire artificielle (2210) configurée pour être implantée à l'intérieur d'un sac capsulaire dans un œil ; et
l'appareil selon l'une quelconque des revendications 1 à 9.

11. Système selon la revendication 10, dans lequel les haptiques (1404a-1404b, 1604a-1604b, 1904a-1904b) de la lentille de contact pseudophake intraoculaire (1400, 1600, 1900) sont plus courtes que des haptiques (2214) de la lentille intraoculaire artificielle (2210).
